# EUROPEAN PATENT SPECIFICATION

(11) **EP 0 200 381 B1**
(45) Date of publication and mention of the grant of the patent: **27.01.1993**
(21) Application number: 86302521.9
(22) Date of filing: 04.04.1986
(51) Int. Cl.: G01N 33/549, G01N 33/545, G01N 33/551, G01N 33/577, G01N 33/564, G01N 33/569, G01N 33/74, G01N 33/70

(54) **A solid phase system for use in ligand-receptor assays**
Festkörpersystem zur Verwendung in Ligand-Rezeptoruntersuchungen
Système à phase solide pour l'utilisation dans des essais du type ligand-récepteur

(30) Priority: 04.04.1985 US 720036
(43) Date of publication of application: 05.11.1986
(60) Divisional application: 91103886.7
(73) Proprietor: HYBRITECH INCORPORATED, San Diego CA 92121 (US)
(72) Inventor: Rubenstein, Albert Samuel, Encinitas, CA 92024 (US)
(74) Representative: Goldin, Douglas Michael

(56) References cited:
- EP-A- 0 119 622
- WO-A-82/02601
- WO-A-85/05451
- FR-A- 2 487 983
- GB-A- 1 420 916
- US-A- 3 951 748
- US-A- 4 338 094
- US-A- 4 444 879

## Description

This invention relates to ligand-receptor assay processes. In another aspect, it relates to a solid phase system for use in ligand-receptor assays, particularly immunoassays using monoclonal antibodies.

This application relates to the subject matter of G. Valkirs et al., US Patent No. 4632901 and US Patent No. 4727019. The disclosures of all these references are incorporated by reference herein.

Ligand-receptor assays, particularly immunoassays, provide sensitive diagnostic tools for the in vitro detection in serum and other body fluids of analytes associated with disease and other physiological conditions of clinical significance.

In the past, immunoassays have typically relied on a polyclonal antibody preparation bound to a solid phase. In such assays, a solution of antigen labeled to permit detection is allowed to compete with antigen in a sample for the solid phase antibody. The extent to which the labeled antigen is bound to the solid phase or is detected in the liquid phase can be used as a measure of the presence and quantity of antigen in the sample being analyzed.

Subsequently, non-competitive immunometric assays became available. In these assays, a polyclonal antibody preparation bound to a solid phase is also used. The sample containing the suspected antigen is allowed to contact the solid phase in order for the antigen to bind to the antibodies on the solid phase. Typically, after an incubation step the sample is separated from the solid phase which is then washed and incubated with a solution of additional polyclonal antibodies which has been labeled, for example with a radionuclide, an enzyme, or a fluorescent moiety, to permit detection.

After this second incubation, the unbound labeled antibody is separated from the solid phase and the amount of labeled antibody in either the liquid phase or bound to the solid phase in an antibody:antigen:antibody sandwich is determined as a measure of the presence and/or concentration of antigen in the sample tested.

More recently, immunoassay processes have been modified to use monoclonal antibodies. For example, U.S. 4,376,110 describes two-site immunometric assays using pairs of monoclonal antibodies, one bound to a solid phase and the other labeled to permit detection. The use of monoclonal antibody pairs which recognize different epitopic sites on an antigen has made it possible to conduct simultaneous immunometric assays in which the antigen and labeled antibody incubations do not require the intermediate steps of prior processes.

In the foregoing immunoassay processes, the solid phase system typically comprises an antibody bound to a bead, or alternatively, an antibody coated on a material such as a membrane or filter, suitable to capture an antigen of interest. At present, the preparation of such solid phase systems characteristically requires activation procedures to facilitate the coating of an antibody to a solid support. Additionally, a backcoating procedure, involving the coating of the solid support with a substance effective to inhibit non-specific binding, is generally required. The activation and backcoating procedures are time-consuming and difficult procedures, and as a result, render the preparation of solid phase systems very costly.

In addition to the above-described limitations associated with the preparation of solid phase systems presently available, it has not been possible to assay a given sample for more than one analyte of interest simultaneously on a single solid phase system. Further, present solid phase systems lack the internal controls (i.e., positive and negative controls) which are essential for a determination of the validity and reliability of an assay. The preparation of solid phase systems for a multiple immunoassay process and/or an internal control system has been, prior to the present invention, very difficult.

EP-A-0119622 describes an integral element for a biological reaction comprising at least two layers consisting essentially of (1) a composite porous biological reaction layer comprising a fibrous material in which is dispersed a particulate material to which an active substance capable of participating in a biochemical reaction is fixed, and (2) a porous layer of a fibrous material. To prepare the two fibrous layers and to obtain the dispersion of particulate material in layer (1) a process analogous to paper making is used involving wet slurries.

US-A-3951748 describes a matrix in which a particulate sorbent, carrier protein molecules bound to receptors, is physically immobilized within the pores of the matrix.

US-A-4338094 describes a rigid capsule, porous to fluids (e.g. made from two die-stamped semi-spheres) in which are encapsulated particles which support antibodies.

Accordingly, there exists a need for a solid phase system which may be prepared more efficiently and which minimizes the difficulty and expense of preparation. Additionally, there exists a need for an improved solid phase system which may be utilized to assay a sample for at least two analytes of interest simultaneously. Further, there exists a need for an improved solid phase system which permits the incorporation of internal controls for the performance of assays.

The present invention provides a solid phase system for use in ligand-receptor assays, particularly immunoassays, for the detection of a selected analyte in a fluid sample. As used herein, the term "ligand-receptor assay" refers to an assay for an analyte which is detected by the formation of a complex between a ligand and another substance capable of specific interaction with the ligand, i.e., receptor. The ligand may be the analyte itself or a substance which if detected can be used to infer the presence of the analyte in a sample. Persons skilled in the art will appreciate that, depending upon the analyte of interest, a member of a specific binding pair may be either receptor or ligand depending upon assay design. In the context of the present invention, the term "ligand" encompasses antigens, haptens, antibodies, deoxyribonucleic acid (DNA), ribonucleic acid (RNA), hormones, metabolites and other naturally occurring substances of diagnostic interest having a specific binding partner therefor, i.e., the receptor of the ligand-receptor assay.

The solid phase system of the invention comprises a porous matrix, such as a membrane or filter, within which microspheres are entrapped. Preferably, the microspheres are entrapped within a discrete or defined zone of the matrix. Additionally, the microspheres, selected to have a size compatible with the pore size of the porous matrix, are bound with a receptor such as an antibody, preferably a monoclonal antibody, antigen, nucleic acid sequence or other substance capable of capturing the selected ligand when exposed to a sample containing the ligand. For example, the microspheres may be bound with an allergen capable of capturing an IgE antibody in a sample which is specific for the bound allergen.

In preferred solid phase systems, distinct groups of microspheres to which are bound an antibody, antigen or other suitable receptor substance are entrapped within discrete zones of the porous matrix so as to permit the performance of a multiple assay for the detection of at least two selected analytes. Further, distinct groups of microspheres may be entrapped within the porous matrix so as to incorporate internal control systems for the detection of selected analytes.

The present invention is further directed to a ligand-receptor assay process comprising, as a first step, the introduction of a fluid sample onto the solid phase system whereby, as the fluid flows through the solid phase system, the receptor bound to the microspheres captures the selected target ligand. Following the addition of the sample, a solution of a receptor conjugate capable of binding the target ligand and labeled so as to permit detection is added to the solid phase system. As used herein the term "receptor conjugate" refers to a complex comprising a receptor and a label capable of detection. In the case of an immunometric assay for a target antigen the receptor conjugate may be a labeled antibody, preferably a monoclonal antibody. Alternatively, if the target ligand is an antibody, labelled antigen may be used as a receptor conjugate. Unbound receptor conjugate may thereafter be removed by a washing step. The presence of bound receptor conjugate on the solid phase system is then determined as an indication of the presence of the analyte in the sample.

This invention has been summarized in order that the drawings and detailed description that follow may be better understood and the contribution to the art may be better appreciated.

Figure 1 is a conceptualization of a porous matrix useful in the present invention and the manner of addition of microspheres thereto.

Figure 2 is a conceptualization of a cross section of a solid phase system of the present invention.

Figure 3 is a top view of a solid phase system of the present invention for the detection of a single analyte in a sample.

Figure 4 is a top view of a preferred solid phase system of the present invention for the multiple detection of different analytes in a sample.

Figure 5 is a top view of a preferred solid phase system of the present invention for the detection of a single analyte in a sample including internal positive and negative controls.

As indicated above, the present invention provides a solid phase system for use in ligand-receptor assays, particularly immunoassays, for the detection of a selected analyte in a fluid sample. In accordance with the present invention, the solid phase system comprises a porous matrix in which microspheres may be entrapped. A variety of porous matrices, including natural and synthetic matrices, may be suitably utilized provided microspheres of a size compatible with the pore size of the matrix may be entrapped in accordance with the invention. Among the matrices preferred for use are membranes or filters comprised of glass fibers, nylon, or ceramic materials having a defined porosity.

The solid phase system further comprises microspheres to which are bound a selected receptor, such as an antibody, preferably a monoclonal antibody, antigen or other suitable receptor substance capable of capturing the ligand of interest. Microspheres comprised of a polymeric material such/as latex polyethylene, polypropylene, or polystyrene are preferred for use in the present invention. However, it will be recognised by those skilled in art that a variety of microspheres, comprised of either natural or synthetic materials, may be utilized. In the case of polymers, the polymer is selected on the basis of its ability to facilitate binding with the selected member of the ligand-receptor pair, e.g., it may be a polymer or copolymer having a functional group which facilitates binding by covalent bond formation or by a coating operation. Additionally, the microspheres are selected to have a size effective to permit their entrapment within the porous matrix as well as the flow of fluid around the microspheres and through the matrix. Preferred for use are microspheres having a size within the range of from about 0.1 to about 50 microns in diameter. We have found that microspheres having a size within this range tend to maximize the effective surface area available for reaction with the target ligand as aggregation and adhesion of the microspheres within the matrix is minimized. Particularly preferred for use are microspheres having a size within the range of from about 0.1 to about 2.0 microns in diameter; microspheres having a size within this range tend to enhance the contact and kinetics for reaction between receptor bound to the microspheres and the target ligand.

In accordance with the present invention, the microspheres selected for use are activated with a suitable receptor, e.g., an antibody, antigen or other biological substance suitable for use as a receptor to bind and capture the target ligand. Activation may be achieved by covalent binding or, in appropriate cases, by application of a coating of the receptor on the microspheres. In the case of an immunoassay to determine the presence of an antigen in a sample, the receptor is preferably a monoclonal antibody, however, polyclonal antibodies from antisera may be suitably used. Techniques for the coating or covalent binding of proteins to microspheres as well as for monoclonal and polyclonal antibody preparation, are well known to the art and require no repetition herein. The activated microspheres are entrapped in the matrix, preferably within a defined zone or zones of the matrix and in a predetermined pattern. Additionally, we have unexpectedly found that it is desirable to use an extremely low concentration of activated microspheres in solution for entrapment, preferably within the range of from about .01 to 1.0%, to provide increased sensitivity in an assay. The means for entrapment of the microspheres within the matrix is not critical and a variety of means may be used. For example, the microspheres may be added to the surface of the porous matrix as a suspension in a suitable liquid vehicle, for example, as a polymer latex, preferably a monodisperse latex, which is subsequently removed in a drying step. Gravity, a vacuum or capillary action may be used to draw the microspheres into discrete zones within the porous matrix prior to removal of the vehicle. As the microspheres are activated prior to entrapment, the present invention overcomes limitations associated with the preparation of the solid phase systems of the prior art.

In accordance with the present invention, the receptor-bound microspheres are preferably entrapped within the matrix in a manner which permits their mobility within the matrix and inhibits their agglutination or aggregation within the matrix, and further, which permits rapid fluid flow through the matrix and around the particles. While applicant does not intend to be bound by any theory, it is believed that the free movement of microspheres within the matrix may enhance the contact between receptor bound to the microspheres and the target ligand and permits effective washing during an assay process. Additionally, as indicated above, to attain the desired sensitivity of an assay process, the number and size of the entrapped microspheres must be optimized within the ranges set forth herein. Further, the microspheres may be coated prior to entrapment to minimize or inhibit their aggregation and adhesion within the matrix.

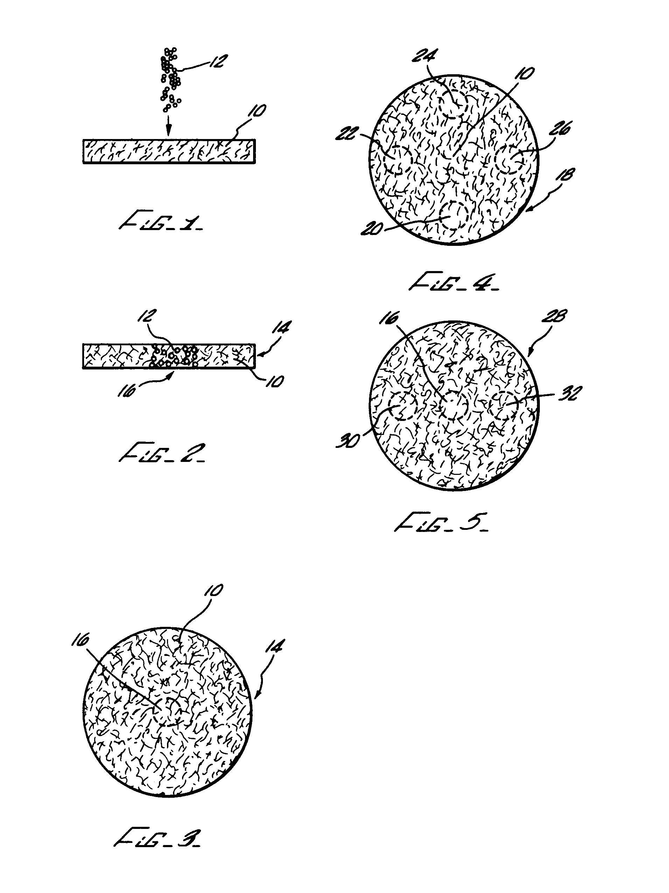

Turning now to Figure 1, there is shown in cross section a conceptualization of a porous matrix 10 useful in the present invention and the manner of addition of activated microspheres 12 thereto (microsphere vehicle is not shown). In Figure 2 there is shown in cross section a conceptualization of a solid phase system 14 of the present invention. Thus, in Figure 2, the porous matrix 10 is shown having the activated microspheres 12 entrapped in a defined or discrete zone 16 within the matrix 10. A top view of the solid phase system 14 for use in an assay for the detection of a single analyte, as conceptualized in Figure 2, is illustrated in Figure 3. Accordingly, in Figure 3, there is shown the discrete zone 16 in which microspheres 12 are entrapped for the capture of a ligand within the matrix 10 of the solid phase system 14.

A preferred solid phase system of the present invention comprises plural groups of microspheres entrapped in discrete zones, preferably in a predetermined pattern, within the matrix. Each group of microspheres is bound, prior to entrapment, with a different receptor, such as an antibody or antigen capable of capturing a different ligand of interest. Accordingly, in one embodiment of the invention, each group of microspheres comprises a population of microspheres bound with the same antibody, antigen or other receptor selected for use in the assay. Alternatively, a group of microspheres may comprise a mixture of microspheres to which are bound different receptors. For example, in the case of an immunoassay for an antigen, each group of microspheres may comprise at least two subpopulations of microspheres wherein each subpopulation is bound with an antibody, preferably a monoclonal antibody, capable of binding with a different determinant or epitope of the antigen. Preferably, the monoclonal antibodies bound with the subpopulations of microspheres comprising a distinct group of microspheres are selected to have a specific reactivity with non-interfering epitopes of the target ligand, thereby enhancing the sensitivity and specificity of the assay.

Accordingly, a solid phase system as described above is useful in a multiple ligand-receptor assay for the simultaneous detection of at least two selected analytes in a given sample. In the case of an immunoassay, the presence of different analytes within a sample may be detected simultaneously by a distinct color reaction for each analyte by the use of different enzyme labeled antibodies which generate distinct color reactions upon addition of a suitable substrate. Alternatively, different groups of microspheres may be entrapped within the porous matrix in a way which permits their identification by reason of their particular location in the matrix.

Further, the preferred solid phase system as described above is particularly useful where it is highly desirable to simultaneously determine the presence of more than one analyte of interest in a sample, such as for the determination of causative agents of an allergic response. This may be readily accomplished by entrapping distinct groups of microspheres, each of which is bound with one of a group of specific allergens (i.e., proteins or carbohydrates which are suspect in eliciting an immune response), within discrete zones of the matrix. Such a solid phase system provides, in essence, a panel of allergens capable of capturing IgE antibodies which may be present in a patient serum sample. Accordingly, the pattern of reactivity on the solid phase system, as determined by the presence of allergen-specific IgE antibodies in a given sample, provides an indication of the allergens eliciting a given allergic response. In such assays, typically the allergen-IgE pairs are detected by using labeled anti-IgE antibody as described in U.S. 3,720,760.

Referring now to Figure 4, there is shown a top view of a preferred solid phase system 18 for use in a multiple assay process in accordance with the conceptualization of a solid phase system 14 in Figure 2. Thus, in Figure 4 there is shown a matrix 10 in which there are discrete zones 20, 22, 24 and 26 comprising groups of microspheres, each of which is activated with a different receptor for the capture of different ligands of interest on the solid phase system 18.

Further in accordance with the present invention, a preferred solid phase system comprises distinct groups of microspheres as an internal control system (i.e., positive and/or negative controls). For example, the microspheres comprising a positive control may be bound with the target ligand or other suitable receptor substance capable of mimicing the binding of the target ligand. By comparison, the microspheres comprising a negative control are either without a bound component or, preferably, bound with a substance, e.g. antibody or antigen or DNA as the case may be, which is incapable of capturing the ligand of interest but which is capable of mimicing the non-specific binding interactions of the microsphere-bound receptor with components of the sample other than the target ligand. Such a solid phase system, which may be utiliized in a single or multiple assay, provides a means for determining the validity and reliability of the assay. Thus, in an immunoassay, the incorporation of a negative control provides a means for determining whether a false positive result, generally attributable to the nonspecific binding of a sample component with the receptor, has occurred. A positive control, on the other hand, reduces the likelihood that a false negative result will go undetected.

Additionally, the present invention provides a means for incorporating internal references for qualitative and quantitative determinations of a target ligand in an assay process as set forth and described in the application of G. Valkirs et al., filed March 21, 1986, assigned to the some assignee as the present invention, and incorporated by reference herein.

Turning now to Figure 5, there is shown a top view of a preferred solid phase system 28 as described above for the detection of a single analyte and which incorporates internal controls. Thus, Figure 5 depicts, Consistent with the conceptualization of Figure 2, a solid phase system 28 having a discrete zone 16 within the matrix 10 comprising microspheres to which the selected receptor is bound. Discrete zone 30 within the matrix 10 comprises microspheres to which the target ligand is bound as a positive control and discrete zone 32 within matrix 10 comprises microspheres which are preferably coated with a substance which mimics the non-specific binding characteristics of the receptor as a negative control. Thus, when system 28 is used in an assay of a patient sample which contains the analyte of interest, both zone 16 and 30 should indicate a positive result. On the other hand, a change in zone 32 corresponding with a change in zone 16 would be interpreted as a false positive result.

As previously indicated, the solid phase system of the present invention are of significant utility in the performance of ligand-receptor assays, particularly multiple ligand-receptor assays for the detection of at least two analytes of interest and/or ligand-receptor assays incorporating an internal control system. While the present invention is particularly useful for the performance of immunoassays, those skilled in the art will appreciate that with suitable modification, the solid phase system provided by the invention may be utilized for other ligand-receptor assays, including assays involving nucleic acid probe technology.

In accordance with the ligand-receptor processes of the invention, a fluid sample suspected of containing the analyte(s) of interest is introduced onto the solid phase system as described above. In the case of an immunoassay, the solid phase preferably incorporates as a receptor a monoclonal antibody bound to the microspheres, although a polyclonal antibody preparation may be used when the ligand of interest is an antigen. If the ligand of interest is an antibody, however, the solid phase may comprise an antigen for which the antibody to be detected is specific. Following the flow of fluid sample through the solid phase system and into the absorbent member, a solution of receptor conjugate, capable of binding with the target ligand and labeled so as to permit detection, is added. In the case of immunoassays, the receptor conjugate may be an antibody, preferably a monoclonal antibody, or antigen capable of binding with the ligand of interest. The addition of receptor conjugate, as appropriate, permits the formation of a complex with the ligand captured by the solid phase system. In the case of an immunoassay, if the microspheres comprising the solid phase system are bound with monoclonal antibody, and the labeled antibody is also a monoclonal antibody, the two antibodies are selected to bind to non-interfering binding sites of the antigen, essentially as described in U.S. 4,376,110 and U.S. 4,486,530, the disclosures of which are incorporated by reference. In presently preferred embodiments, the receptor conjugate is labeled with an enzyme, however other conventional labels, such as radionuclides, fluorescent agents, phosphorescent agents and polymers containing dyes or chemiluminescent moieties may be suitably utilized. After the solution of receptor conjugate has flowed through the solid phase system, a washing solution may be added to remove unbound receptor conjugate. Thereafter, the receptor conjugate complexed with the ligand of interest is detected. If an enzyme has been selected as the label component, the bound receptor conjugate is detected by the addition of an appropriate enzyme substrate to the solid phase system. Upon reaction with the substrate, the enzyme will generate, if properly selected, a color change on the solid phase system which may be detected by visual or instrumental means.

It will be recognized by those skilled in the art that assays for a broad range of analytes may be performed in accordance with the present invention. The list of potential analytes of interest is too lengthy for incorporation herein. However, antigens such as prostatic acid phosphatase, prostate-specific antigen, alphafetoprotein, carcinoembryonic antigen, leutenizing hormone, creatine kinase isoenzimes and other antigens in serum, plasma, urine or other biological fluids may be detected in an immunoassay. Additionally, the present invention is useful for the assay of viruses, bacteria, parasites or fungi, or antigens or antibodies associated therewith, including, for example, Rubella virus, Rota virus, adeno virus, respiratory syncitial virus, HTLV, hepatitis virus, hepatitis/A, hepatitis/B, hepatitis nonA nonB, influenza virus, cytomegalovirus and herpes virus, as well as group A and group B streptococcus, Neisseria gonorrhea, Trichomonas vaginalis, Candida albicans, Chlamydia trachomatis and Hemophilus influenza.

Further, it will be appreciated by those skilled in the art having the benefit of this disclosure that the present invention is applicable to assays involving nucleic acid probe technology. Specifically, a nucleic acid sequence complementary to a portion of the nucleic acid sequence of a ligand of interest may be bound to microspheres which are thereafter entrapped in a porous matrix. Such a solid phase system may be utilized in assay processes. To perform such assay processes, a fluid sample suspected of containing the target ligand is introduced onto the solid phase and the ligand of interest is captured on the solid phase. Thereafter, a labeled nucleic acid probe having a nucleic acid sequence complementary to a different portion of the nucleic acid sequence of the target ligand is added to permit the formation of a complex of the labeled nucleic acid probe and the ligand captured on the solid phase. The detection of the labeled nucleic acid probe bound to the solid phase system provides an indication of the presence of the analyte in a given sample.

The following examples demonstrate the preparation of a solid phase system comprising microspheres entrapped within a glass fiber filter and the use of such a solid phase system to perform an immunoassay in accordance with the present invention. The examples are presented solely for purposes of illustration are not intended to limit the present invention in any way.

### EXAMPLE I

### DETECTION OF ANTIGEN

The assay set forth in the following example is an immunoassay for the detection of human choriogonadotripin (HCG), an antigen present in elevated levels in the urine of pregnant women.

### Activation of Microspheres

A monodisperse layer of polystyrene microspheres, 0.8 microns in diameter (Interfacial Dynamics Corporation, Portland, Oregon) were selected and activated by passive absorption of a preparation of monoclonal antibody derived from hybrid cell line HCU 061 (Hybritech Incorporated, San Diego, CA), specific for the HCG antigen. The microsphere size was selected to be compatible with Whatman GF/F glass fiber filter (Whatman Company, Clifton, New Jersey) by preliminary experimentation using fluorescent-labeled microspheres to determine retention within the filter. The monoclonal antibodies were obtained from ascites fluid and purified by Na₂SO₄ precipitation and DEAE ion exchange chromatography using standard methodology.

The antibody-bound microspheres were then backcoated with a 1% solution of Bovine Serum Albumin (BSA) in 50 mM phosphate (pH 8.0) to reduce non-specific binding of HCG and labeled antibody, following conventional procedures.

### Entrapment of Microspheres

A 10 µl quantity of the activated microspheres were resuspended in 50 mM phosphate (pH 8.0) at 0.25% w/v concentration and pipetted onto the Whatman GF/F glass fiber filter incorporated in an immunoconcentration apparatus as shown in Figure 6. The microspheres were thereafter allowed to settle within the filter in a discrete circular zone at the center of the filter. The filter was allowed to air dry for 15 minutes.

### Detection of Antigen

Approximately 250 µl of urine containing 125 mIU/ ml HCG were applied to the filter. Approximately 100 µl of a preparation of a second monoclonal antibody against HCG, derived from hybrid cell line HCO 514, and labeled with the enzyme alkaline phosphatase, was then added. After a 5 minute incubation during which time the enzyme-labeled antibody conjugate was drawn through the filter, the filter was washed with 2ml of 10mM Tris-buffered saline (pH 8.0). Approximately 100 µl of a solution containing indoxyl phosphate, a substrate for the enzyme label was then added. After 5 minutes of incubation the filter was observed visually for a color reaction.

A dark blue color developed in the discrete zone of the filter where the HCU-061 activated microspheres were entrapped, indicating the presence of HCG antigen.

### EXAMPLE II

### ANTIBODY DETECTION

Antibodies to Rubella virus were detected in a manner similar to that described above for detection of HCG antigen in Example I. Microspheres of 0.8 microns in diameter which had been activated with purified Rubella antigen were entrapped in a discrete zone on the Whatman GF/F glass fiber filter incorporated in an immunoconcentration apparatus as shown in Figure 6. As a negative control, a group of non-antigen coated microspheres were entrapped in an adjacent zone on the filter.

### Detection of Antibodies

A 100 µl quantity of serum predetermined to be positive for the presence of antibodies to Rubella virus was applied onto a second filter prepared as described above. Simultaneously, a 100 µl quantity of serum predetermined to be negative for the presence of anti-bodies on each filter with 2 ml of 10mM Tris-buffered saline (pH 8.0), 100 µl of enzyme conjugated-antibodies against human IgG (Kirkegaard & Perry Laboratories, Inc., Gaithersburg, Maryland) were added and allowed to react with the human IgG complexed with the Rubella antigen bound to the microspheres. After washing with 2 ml of 10 mM Tris-buffered saline (pH 8.0) to remove unbound conjugate, substrate for the enzyme label was added to detect the presence of any antigen-antibody complexes formed.

The positive sera resulted in a blue color in the discrete zone of the filter where microspheres bound with Rubella antigen were entrapped. (In the zone of the negative control, no color appeared.) The negative sera did not produce a color reaction.

It will be appreciated that the sensitivity of the assays of the examples may be adjusted by varying the volume and concentration of the microspheres, or incubation time, conjugate concentration, and other parameters commonly used in assay procedures.

## Claims

1. A solid phase system for use in a ligand-receptor assay for the detection of a selected analyte in a fluid sample, comprising a porous matrix in which microspheres are entrapped wherein said microspheres are bound with a receptor capable of capturing a target ligand.

2. The solid phase system according to Claim 1 wherein said matrix is a membrane or filter in which microspheres of a size compatible with the pore size of said matrix may be entrapped.

3. The solid phase system according to Claim 2 wherein said microspheres are selected to have a size which maximizes the effective surface area of said microspheres within said matrix.

4. The solid phase system according to Claim 2 wherein said microspheres are selected to have a size within the range of from about 0.1 micron to about 50 microns in diameter.

5. The solid phase system according to Claim 2 wherein said microspheres are selected to have a size within the range of from about 0.1 micron to about 2.0 microns in diameter.

6. The solid phase system according to Claim 1 wherein said microspheres are entrapped within said matrix in a manner to permit the mobility of said microspheres/within said matrix and inhibit the aggregation or adhesion of said microspheres within said matrix

7. The solid phase system according to Claim 2 wherein said membrane or filter is of a material selected from glass fibers, nylon or ceramic materials.

8. The solid phase system according to Claim 1 wherein said microspheres are entrapped within a discrete zone of said matrix.

9. The solid phase system according to Claim 1 wherein said microspheres are comprised of a polymeric material capable of being bound with said receptor.

10. The solid phase system according to Claim 9 wherein said polymeric material is selected from/latex, polyethylene, polypropylene and polystryrene and copolymers thereof.

11. The solid phase system according to Claim 1 wherein said ligand-receptor assay is an immunoassay.

12. The solid phase system according to Claim 11 wherein said receptor bound with said microspheres is an antibody or antigen capable of capturing said target ligand.

13. The solid phase system according to Claim 12 wherein said antibody is a monoclonal antibody.

14. The solid phase system for use in a multiple ligand-receptor assay for the simultaneous detection of at least two selected analytes in a fluid sample comprising a porous matrix in which distinct groups of microspheres are entrapped within discrete zones wherein each distinct group of microspheres is bound with a receptor capable of capturing a different target ligand.

15. The solid phase system according to Claim 14 wherein said matrix is a membrane or filter in which microspheres of a size compatable with the pore size of said matrix may be entrapped.

16. The solid phase system according to Claim 15 wherein said membrane or filter is of a material selected from glass fibers, nylon or ceramic materials.

17. The solid phase system according to Claim 14 wherein said microspheres are comprised of a polymeric material capable of being bound with said receptor.

18. The solid phase system according to Claim 17 wherein said polymeric material is selected from latex, polyethylene, polypropylene and polystyrene and copolymers thereof.

19. The solid phase system according to Claim 14 wherein said ligand-receptor assay is an immunoassay.

20. The solid phase system according to Claim 19 wherein said receptor bound with at least one group of microspheres is an antibody or antigen capable of capturing said target ligand.

21. The solid phase system according to Claim 20 wherein said antibody is a monoclonal antibody.

22. The solid phase system according to Claim 21 wherein said group of microspheres comprises at least two sub-populations of microspheres to which are bound monoclonal antibodies selected to bind at non-interfering epitopes of said target ligand.

23. A solid phase system for use in a ligand-receptor assay for the detection of at least one selected analyte in a fluid sample comprising a porous matrix in which distinct groups of microspheres are entrapped within discrete zones wherein:
a) at least one group of microspheres is bound with a receptor capable of capturing a target ligand; and
b) at least one group of microspheres is bound with said target ligand or other suitable receptor substance as a positive control for the detection of said analyte.

24. A solid phase system for use in a ligand-receptor assay for the detection of at least one selected analyte in a fluid sample comprising a porous matrix in which distinct groups of microspheres are entrapped within discrete zones wherein:
a) at least one group of microspheres is bound with a receptor capable of capturing a target ligand; and
b) at least one group of microspheres is bound with a substance incapable of capturing said target ligand, or without a bound component, as a negative control for the detection of said analyte.

25. The solid phase system according to Claims 23 or 24 wherein said matrix is a membrane or filter in which microspheres of a size compatible with the pore size of said matrix may be entrapped.

26. The solid phase system according to Claims 25 or 24 wherein said membrane or filter is of a material selected from glass fibers, nylon or ceramic materials.

27. The solid phase system according to Claims 23 or 24 wherein said microspheres are comprised of a polymeric material capable of being bound with said receptor.

28. The solid phase system according to Claim 27 wherein said polymeric material is selected from/latex, polyethylene, polypropylene and polystyrene and copolymers thereof.

29. The solid phase system according to Claims 23 or 24 wherein said ligand-receptor assay is an immunoassay.

30. The solid phase system according to Claim 29 wherein said receptor bound with at least one group of microspheres is an antibody or antigen capable of capturing said target ligand.

31. The solid phase system according to Claim 30 wherein said antibody is a monoclonal antibody.

32. The solid phase system according to Claim 30 further comprising a distinct group of microspheres to which is bound the target ligand or other suitable receptor substance as a positive control.

33. The solid phase system according to Claim 1 wherein said assay is a nucleic acid probe assay and wherein said receptor comprises a nucleic acid sequence complementary to a portion of the nucleic acid sequence of said target ligand.

34. The solid phase systems according to claims 10, 18 or 28 wherein said microspheres are comprised of latex.

## Patentansprüche

1. Festphasensystem zur Verwendung in einem Bestimmungsverfahren mit Ligandenrezeptoren zum Erfassen eines ausgewählten Analyten in einer Fluidprobe, umfassend eine poröse Matrix, in die Mikrobereiche eingeschlossen sind, wobei die Mikrobereiche mit einem Rezeptor verbunden sind, der zum Einfangen eines Ziellganden befähigt ist.

2. Festphasensystem nach Anspruch 1, wobei die Matrix eine Membran oder ein Filter ist, in welche(s) Mikrobereiche einer Größe eingeschlossen werden können, die mit der Porengröße der Matrix verträglich ist.

3. Festphasensystem nach Anspruch 2, wobei die Mikrobereiche so ausgewählt sind, daß sie eine Größe aufweisen, die die wirksame Oberfläche der Mikrobereiche innerhalb der Matrix maximiert.

4. Festphasensystem nach Anspruch 2, wobei die Mikrobereiche so ausgewählt sind, daß sie eine Durchmessergröße im Bereich von etwa 0,1 Mikron bis etwa 50 Mikron aufweisen.

5. Festphasensystem nach Anspruch 2, wobei die Mikrobereiche so ausgewählt sind, daß sie eine Durchmessergröße im Bereich von etwa 0,1 Mikron bis etwa 2,0 Mikron aufweisen.

6. Festphasensystem nach Anspruch 1, wobei die Mikrobereiche in der Matrix auf eine Weise eingeschlossen sind, daß sie Beweglichkeit der Mikrobereiche innerhalb der Matrix gestattet und die Aggregation oder Adhesion der Mikrobereiche innerhalb der Matirx unterbunden ist.

7. Festphasensystem nach Anspruch 2, wobei die Membran oder das Filter ein Material darstellt, welches ausgewählt ist aus Glasfasern, Nylon oder Keramikmaterialen.

8. Festphasensystem nach Anspruch 1, wobei die Mikrobereiche innerhalb einer diskreten Zone der Matrix eingeschlossen sind.

9. Festphasensystem nach Anspruch 1, wobei die Mikrobereiche ein polymeres Material aufweisen, welches zum Eingehen einer Bindung mit dem Rezeptor befähigt ist.

10. Festphasensystem nach Anspruch 9, wobei das polymere Material ausgewählt ist aus Latex, Polyethylen, Polypropylen und Polystyrol und Copolymeren davon.

11. Festphasensystem nach Anspruch 1, wobei das Bestimmungsverfahren mit Ligandenrezeptor ein Immunoassay ist.

12. Festphasensystem nach Anspruch 11, wobei der mit den Mikrobereichen verbundene Rezeptor ein Antikörper oder ein Antigen ist, welcher(s) zum Einfangen des Zielliganden befähigt ist.

13. Festphasensystem nach Anspruch 12, wobei der Antikörper ein monoklonaler Antikörper ist.

14. Festphasensystem zum Einsatz in einem Vielfach-Bestimmungsverfahren mit Ligandenrezeptoren zum gleichzeitigen Erfassen von mindestens zwei ausgewählten Analyten in einer Fluidprobe, umfassend eine poröse Matrix, in die innerhalb diskreter Zonen unterschiedliche Gruppen von Mikrobereichen eingeschlossen sind, wobei jede der unterschiedlichen Gruppen von Mikrobereichen mit einem Rezeptor verbunden ist, der zum Einfangen eines anderen Zielliganden befähigt ist.

15. Festphasensystem nach Anspruch 14, wobei die Matrix eine Membran oder ein Filter ist, in welche(s) Mikrobereiche einer Größe eingeschlossen werden können, die mit der Porengröße der Matrix verträglich ist.

16. Festphasensystem nach Anspruch 15, wobei die Membran oder das Filter ein Material darstellt, welches ausgewählt ist aus Glasfasern, Nylon oder Keramikmaterialen.

17. Festphasensystem nach Anspruch 14, wobei die Mikrobereiche ein polymeres Material aufweisen, welches zum Eingehen einer Bindung mit dem Rezeptor befähigt ist.

18. Festphasensystem nach Anspruch 17, wobei das polymere Material ausgewählt ist aus Latex, Polyethylen, Polypropylen und Polystyrol und Copolymeren davon.

19. Festphasensystem nach Anspruch 14, wobei das Bestimmungsverfahren mit Ligandenrezeptor ein Immunoassay ist.

20. Festphasensystem nach Anspruch 19, wobei der mit mindestens einer Gruppe von Mikrobereichen verbundene Rezeptor ein Antikörper oder ein Antigen ist, welcher(s) zum Einfangen des Zielliganden befähigt ist.

21. Festphasensystem nach Anspruch 20, wobei der Antikörper ein monoklonaler Antikörper ist.

22. Festphasensystem nach Anspruch 21, wobei die Gruppe von Mikrobereichen mindestens zwei Unterpopulationen von Mikrobereichen umfaßt, mit der monoklonale Antikörper verbunden sind, die so ausgewählt sind, daß sie sich an nicht interferierende Epitope des Zielliganden anhängen.

23. Festphasensystem zur Verwendung in einem Bestimmungsverfahren mit Ligandenrezeptoren zum Erfassen mindestens eines ausgewählten Analyten in einer Fluidprobe, umfassend eine poröse Matrix, in die innerhalb diskreter Zonen bestimmte Gruppen von Mikrobereichen eingeschlossen sind, wobei
a) mindestens eine Gruppe von Mikrobereichen mit einem Rezeptor verbunden ist, der zum Einfangen eines Zielliganden befähigt ist; und
b) als positive Kontrolle für das Erfassen des Analyten mindestens eine Gruppe von Mikrobereichen mit dem Zielliganden oder einer anderen geeigneten Rezeptorsubstanz verbunden ist.

24. Festphasensystem zur Verwendung in einem Bestimmungsverfahren mit Ligandenrezeptoren zum Erfassen mindestens eines ausgewählten Analyten in einer Fluidprobe, umfassend eine poröse Matrix, in die innerhalb diskreter Zonen bestimmte Gruppen von Mikrobereichen eingeschlossen sind, wobei
a) mindestens eine Gruppe von Mikrobereichen mit einem Rezeptor verbunden ist, der zum Einfangen eines Zielliganden befähigt ist; und
b) als negative Kontrolle für das Erfassen des Analyten mindestens eine Gruppe von Mikrobereichen mit einer Substanz verbunden ist, die zum Erfassen des Zielliganden nicht befähigt ist, oder ohne gebundene Komponente ist.

25. Festphasensystem nach Anspruch 23 oder 24, wobei die Matrix eine Membran oder ein Filter ist, in welche(s) Mikrobereiche einer Größe eingeschlossen werden können, die mit der Porengröße der Matrix verträglich ist.

26. Festphasensystem nach Anspruch 25, wobei die Membran oder das Filter ein Material darstellt, welches ausgewählt ist aus Glasfasern, Nylon oder Keramikmaterialen.

27. Festphasensystem nach Anspruch 23 oder 24, wobei die Mikrobereiche ein polymeres Material aufweisen, welches zum Eingehen einer Bindung mit dem Rezeptor befähigt ist.

28. Festphasensystem nach Anspruch 27, wobei das polymere Material ausgewählt ist aus Latex, Polyethylen, Polypropylen und Polystyrol und Copolymeren davon.

29. Festphasensystem nach Anspruch 23 oder 24, wobei das Bestimmungsverfahren mit Ligandenrezeptor ein Immunoassay ist.

30. Festphasensystem nach Anspruch 29, wobei der mit mindestens einer Gruppe von Mikrobereichen verbundene Rezeptor ein Antikörper oder ein Antigen ist, welcher(s) zum Einfangen des Zielliganden befähigt ist.

31. Festphasensystem nach Anspruch 30, wobei der Antikörper ein monoklonaler Antikörper ist.

32. Festphasensystem nach Anspruch 30, weiter aufweisend eine bestimmte Gruppe von Mikrobereichen, an die der Zielligand oder eine andere geeignete Rezeptorsubstanz als positive Kontrolle gebunden ist.

33. Festphasensystem nach Anspruch 1, wobei das Bestimmungsverfahren ein Verfahren zum Bestimmen von Nukleinsäuren ist, und wobei der Rezeptor eine Nukleinsäuresequenz aufweist, die komplementär zu einem Teil der Nukleinsäuresequenz des Zielliganden ist.

34. Festphasensystem nach Anspruch 10, 18 oder 28, wobei die Mikrobereiche Latex aufweisen.

## Revendications

1. Système à phase solide destiné à être utilisé dans un test ligand-récepteur, pour la détection d'un analyte sélectionné dans un échantillon fluide, comprenant une matrice poreuse dans laquelle sont incluses des microsphères, ledites microsphères étant liées à un récepteur capable de capturer un ligand cible.

2. Système à phase solide selon la revendication 1, dans lequel ladite matrice est une membrane ou un filtre dans laquelle des microsphères, ayant une taille compatible avec les tailles des pores de ladite matrice, peuvent être incluses.

3. Système à phase solide selon la revendication 2, dans lequel lesdites microsphères sont sélectionnées de façon à présenter une taille qui permet d'obtenir une superficie efficace maximale desdites microsphères dans ladite matrice.

4. Système à phase solide selon la revendication 2, dans lequel lesdites microsphères sont sélectionnées de façon à présenter une taille dans la gamme d'environ 0,1 micron à environ 50 microns de diamètre.

5. Système à phase solide selon la revendication 2, dans lequel lesdites microsphères sont sélectionnées de façon à présenter une taille dans la gamme d'environ 0,1 micron à environ 2,0 microns de diamètre.

6. Système à phase solide selon la revendication 1, dans lequel lesdites microsphères sont incluses dans ladite matrice de manière à permettre la mobilité desdites microsphères à l'intérieur de ladite matrice et à empêcher l'agrégation ou l'adhérence desdites microsphères à l'intérieur de ladite matrice.

7. Système à phase solide selon la revendication 2, dans lequel ladite membrane ou filtre est constituée d'un matériau choisi parmi les fibres de verre, le nylon ou les matériaux céramiques.

8. Système à phase solide selon la revendication 1, dans lequel lesdites microsphères sont incluses dans une zone discrète de ladite matrice.

9. Système à phase solide selon la revendication 1, dans lequel lesdites microsphères comprennent un matériau polymère capable d'être fixé audit récepteur.

10. Système à phase solide selon la revendication 9, dans lequel ledit matériau polymère est choisi parmi le latex, le polyéthylène, le polypropylène et le polystyrène, ainsi que leurs copolymères.

11. Système à phase solide selon la revendication 1, dans lequel ledit test ligand-récepteur est un dosage immunologique.

12. Système à phase solide selon la revendication 11, dans lequel ledit récepteur lié auxdites microsphères est un anticorps ou un antigène capable de capturer ledit ligand cible.

13. Système à phase solide selon la revendication 12, dans lequel ledit anticorps est un anticorps monoclonal.

14. Système à phase solide destiné à être utilisé dans un test ligand-récepteur multiple, pour la détection simultanée d'au moins deux analytes sélectionnés dans un échantillon fluide, comprenant une matrice poreuse dans laquelle sont inclus des groupes distincts de microsphères à l'intérieur de zones discrètes, chaque groupe distinct de microsphères étant lié à un récepteur capable de capturer un ligand cible différent.

15. Système à phase solide selon la revendication 14, dans lequel ladite matrice est une membrane ou filtre dans laquelle on peut inclure des microsphères ayant une taille compatible avec la taille des pores de ladite matrice.

16. Système à phase solide selon la revendication 15, dans lequel ladite membrane ou filtre est constituée d'un matériau choisi parmi les fibres de verre, le nylon ou les matériaux céramiques.

17. Système à phase solide selon la revendication 14, dans lequel lesdites microsphères sont constituées d'un matériau polymère capable d'être fixé audit récepteur.

18. Système à phase solide selon la revendication 17, dans lequel ledit matériau polymère est choisi parmi le latex, le polyéthylène, le polypropylène et le polystyrène, ainsi que leurs copolymères.

19. Système à phase solide selon la revendication 14, dans lequel ledit test ligand-récepteur est un dosage immunologique.

20. Système à phase solide selon la revendication 19, dans lequel ledit récepteur, lié à au moins un groupe de microsphères, est un anticorps ou un antigène capable de capturer ledit ligand cible.

21. Système à phase solide selon la revendication 20, dans lequel ledit anticorps est un anticorps monoclonal.

22. Système à phase solide selon la revendication 21, dans lequel ledit groupe de microsphères comprend au moins deux sous-populations de microsphères auquelles sont liés des anticorps monoclonaux choisis pour se lier à des déterminants antigéniques non interférants dudit ligand cible.

23. Système à phase solide destiné à être utilisé dans un test ligand-récepteur, pour la détection d'au moins un analyte sélectionné dans un échantillon fluide, comprenant une matrice poreuse dans laquelle des groupes distincts de microsphères sont inclus dans des zones discrètes, dans lequel :
a) au moins un groupe de microsphères est fixé à un récepteur capable de capturer un ligand cible ; et
b) au moins un groupe de microsphères est fixé audit ligand cible ou à une autre substance réceptrice appropriée, comme témoin positif pour la détection dudit analyte.

24. Système à phase solide destiné à être utilisé dans un test ligand-récepteur, pour la détection d'au moins un analyte sélectionné dans un échantillon fluide, comprenant une matrice poreuse dans laquelle des groupes distincts de microsphères sont inclus dans des zones discrètes, dans lequel :
a) au moins un groupe de microsphères est fixé à un récepteur capable de capturer un ligand cible ; et
b) au moins un groupe de microsphères est fixé à une susbtance incapable de capturer ledit ligand cible, ou sans composant fixé, comme témoin négatif pour la détection dudit analyte.

25. Système à phase solide selon la revendication 23 ou 24, dans lequel ladite matrice est une membrane ou un filtre dans laquelle on peut inclure des microsphères ayant une taille compatible avec la taille des pores de ladite matrice.

26. Système à phase solide selon la revendication 25 ou 24, dans lequel ladite membrane ou filtre est constituée d'un matériau choisi parmi les fibres de verre, le nylon ou les matériaux céramiques.

27. Système à phase solide selon la revendication 23 ou 24, dans lequel lesdites microsphères sont constituées d'un matériau polymère capable d'être fixé audit récepteur.

28. Système à phase solide selon la revendication 27, dans lequel ledit matériau polymère est choisi parmi le latex, le polyéthylène, le polypropylène et le polystyrène, ainsi que leurs. copolymères.

29. Système à phase solide selon la revendication 23 ou 24, dans lequel ledit test ligand-récepteur est un dosage immunologique.

30. Système à phase solide selon la revendication 29, dans lequel ledit récepteur, lié à au moins un groupe de microsphères, est un anticorps ou un antigène capable de capturer ledit ligand cible.

31. Système à phase solide selon la revendication 30, dans lequel ledit anticorps est un anticorps monoclonal.

32. Système à phase solide selon la revendication 30, comprenant en outre un groupe distinct de microsphères auxquelles est fixé le ligand cible ou une autre substance réceptrice appropriée comme témoin positif.

33. Système à phase solide selon la revendication 1, dans lequel ledit test est un test utilisant une sonde d'acide nucléique et dans lequel ledit récepteur comprend une séquence d'acide nucléiques qui est complémentaire avec la partie de la séquence d'acides nucléiques dudit ligand cible.

34. Système à phase solide selon les revendications 10, 18 ou 28, dans lequel lesdites microsphères comprennent du latex.
